# EUROPEAN PATENT SPECIFICATION

(11) **EP 2 427 055 B1**
(45) Date of publication and mention of the grant of the patent: **26.02.2014**
(21) Application number: 10717977.2
(22) Date of filing: 04.05.2010
(51) Int. Cl.: A01N 43/22, A01P 7/04

(54) **SPINETORAM FOR USE IN CONTROLLING FLEA INFESTATIONS IN CAT AND DOG**
SPINETORAM ZUR BEKÄMPFUNG VON FLOHBEFALL BEI KATZEN UND HUNDEN
SPINETORAM POUR LE CONTRÔLE DES INFESTATIONS PAR LES PUCES CHEZ CHAT ET CHIEN

(30) Priority: 08.05.2009 US 176558 P
(43) Date of publication of application: 14.03.2012
(73) Proprietor: Eli Lilly and Company, Indianapolis, IN 46285 (US)
(72) Inventor: SNYDER, Daniel, Earl, Indianapolis, Indiana 46285 (US); WHITE, William, Hunter, Indianapolis, Indiana 46285 (US)
(74) Representative: Bassinder, Emma Marie
(86) International application number: PCT/US2010/033458
(87) International publication number: WO 2010/129491

(56) References cited:
- EP-A1- 2 033 518
- US-A1- 2007 104 750
- US-B1- 6 664 237
- Elanco Animal Health, A Division of Eliy Lilly and Company, Lilly Corporate Center, Indianapolis, IN 46285, U.S.A.: "COMFORTIS (spinosad) Chewable Tablets" INTERNET CITATIONno. PA9181DEAMP, 1 August 2007 (2007-08-01), XP007913924 Retrieved from the Internet: URL:http://www.elancopetprogram.com/pdf/co mfortis-product-label.pdf [retrieved on 2010-07-14]
- THOMAS C SPARKS ET AL: "Neural network-based QSAR and insecticide discovery: spinetoram" JOURNAL OF COMPUTER-AIDED MOLECULAR DESIGN, KLUWER ACADEMIC PUBLISHERS, DO, vol. 22, no. 6-7, 15 March 2008 (2008-03-15), pages 393-401, XP019606742 ISSN: 1573-4951
- KE-XUE HUANG ET AL: "Recent advances in the biochemistry of spinosyns" APPLIED MICROBIOLOGY AND BIOTECHNOLOGY, SPRINGER, BERLIN, DE, vol. 82, no. 1, 10 December 2008 (2008-12-10), pages 13-23, XP019705431 ISSN: 1432-0614

## Description

Ectoparasites such as fleas, lice, blowflies, ticks and mites are problematic for man and animal alike. Such pests seriously impact productivity in the domesticated animal industry by reducing weight gain, causing poor quality hide, wool, and meat, and in some cases resulting in death. Ectoparasites also cause disease and discomfort in companion animals. Ectoparasites are known to carry bacteria and viruses which are pathogenic to humans. The diseases which ectoparasites cause include malaria, lymphatic filariasis, trachoma, trypanosomiasis, and river blindness, for example.

Efforts for controlling ectoparasites have included the use of insecticides and pesticides. For example, spinosyns, which are naturally derived fermentation products, have been employed as ectoparasiticides in companion animals. (Snyder, US 6,664,237).

Derivatives of spinosyns have been employed in agricultural applications. (DeAmicis et al., US 6,001,981). Spinetoram is the common name for a mixture of 25-90%, preferably 50-90% (2R,3aR,5aR,5bS,9S,13S,14R,16aS,16bR)-2-(6-deoxy-3-O-ethyl-2,4-di-O-methy-1-.alpha.-L-mannopyranosyloxy)-13-[(2R,5S,6R)-5-(dimethylamino)tetrahydro--6-methylpyran-2-yloxy]-9-ethyl-2,3,3a,4,5,5a,5b,6,9,10,11,12,13,14,16a,16b--hexadecahydro-14-methyl-1H-as-indaceno[3,2-d]oxacyclododecine-7,15-dione (referred to as "dihydro-Et-J", formula I below), and 10-75%, preferably10-50% (2R,3aR,5aS,5bS,9S,13S,14R,16aS,16bS)-2-(6-deoxy-3-O-ethyl-2,4-di-O-methy- 1-.alpha.-L-mannopyranosyloxy)-13-[(2R,5S,6R)-5-(dimethylamino)tetrahydro--6-methylpyran-2-yloxy]-9-ethyl-2,3,3a,5a,5b,6,9,10,11,12,13,14,16a,16b-tet-radecahydro-4,14-dimethyl-1H-as-indaceno[3,2-o]oxacyclododecine-7,15-dione (referred to as "Et-L", formula II below).

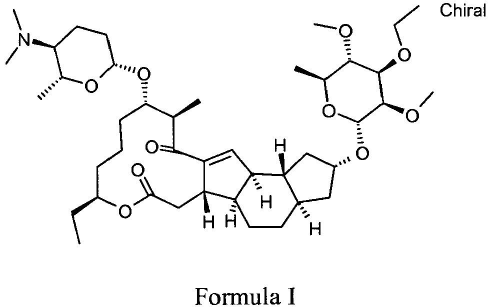

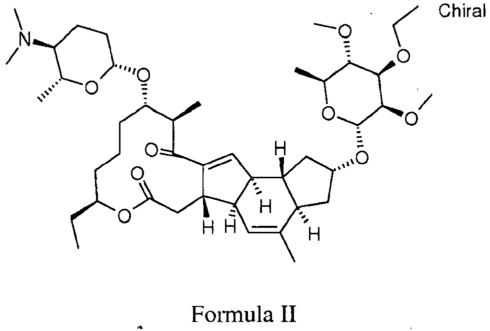

(Podhorez et al., US 2008/0108800A1). Spinetoram is described as providing long-lasting control of a broad spectrum of insect pests in a variety of crops (Dow AgroSciences Spinetoram Technical Bulletin, November 2006). It has been reported spinetoram has been registered in New Zealand as an insecticide in the pome fruit market ("Dow AgroSciences Receives First Global Registration for Spinetoram Insecticide," Dow AgroSciences Newsroom, Corporate News, August 10,2007).

While the use of spinosyns and other insecticides and pesticides have been beneficial, alternative or improved formulations and uses are needed. Desirable formulations and uses would not only provide alternative therapies, but would also overcome at least some limitations of current therapies. Such limitations include toxicity and safety, efficacy (potency and duration), and resistance issues. Also impacting the beneficial use of insecticides and pesticides are administration obstacles, which include mode and recurrence of administration. For example, reducing the frequency of administration while maintaining efficacy is desirable, as dosing animals is often inconvenient and/or difficult. The present invention encompasses ectoparasiticidal formulations for use in animals which provide alternative options for combating ectoparasiticite infestations. Further, they overcome at least some limitations in the use of current insecticides and pesticides, particularly in providing effective long term, safe, systemic control of ectoparasites.

The invention provides spinetoram, or a pharmaceutically acceptable salt thereof, for use in controlling flea infestations on a dog or cat, wherein the spinetoram is to be systemically administered by oral administration. The invention also provides a single or pulse dose formulation for systemically controlling ectoparasite infestations on a dog or cat comprising spinetoram, or a pharmaceutically acceptable salt thereof, and a pharmaceutically acceptable carrier in an oral dosage form selected from a tablet, capsule, or liquid at a dose of 10 to 60 mg of spinetoram or a pharmaceutically acceptable salt thereof per kg of body weight of the dog or cat. Another aspect of the therapeutic uses and formulations using spinetoram is the ability to provide prolonged systemic control of ectoparasite infestations, thus decreasing the recurrence of dosing an animal, such as no more than every one or two weeks, or every month or more.

The host animal may be a mammal or non-mammal, such as a bird (turkeys, chickens) or fish. Where the host animal is a mammal, it may be a human or non-human mammal. Non-human mammals include domestic animals, such as livestock animals and companion animals. Livestock animals include cattle, camellids, pigs, sheep, goats, and horses. Companion animals include dogs, rabbits, cats, and other pets owned and maintained in close association with humans as part of the human-animal bond.

Ectoparasites include insect and acarine pests which commonly infest or infect animals, and include the egg, larval, pupal, nymphal, and adult stages thereof. Such pests include fleas, lice, mosquitoes, mites, ticks, and blood-sucking, biting or nuisance fly species. A particular target is fleas, and more particularly *Ctenocephcalides felis.*

"Controlling" refers to either ameliorating or eliminating a current infestation, or preventing an infestation, in an animal host.

"Effective amount" refers to the amount of spinetoram, or a salt thereof, sufficient to control an ectoparasite, and includes causing a measurable reduction in the ectoparasite infestation population. This control may be the result of spinetoram or its conjugate or salt entering the system of the pest when it feeds, or through a repellant or in vivo action due to the systemic presence of spinetoram or its conjugate or salt thereof. Ranges for spinetoram or a salt thereof in the therapeutic uses and formulations range from 0.01 to 1000 mg/kg, more desirably, 0.1 to 100 mg/kg, and particularly desirable, 10 to 60 mg/kg of body weight of the animal.

"Pharmaceutically acceptable" as used in this application, for example with reference to salts and formulation components such as carriers, includes "veterinarily acceptable", and thus includes both human and animal applications independently.

Pharmaceutically acceptable salts, and common methodology for preparing them are known in the art. *See, e.g.,* P. Stahl, et al., HANDBOOK OF PHARMACEUTICAL SALTS: PROPERTIES, SELECTION AND USE, (VCHA/Wiley-VCH, 2002); S.M. Berge, et al., "Pharmaceutical Salts," Journal of Pharmaceutical Sciences, Vol. 66, No.1, January 1977. Examples of salts include salts formed by standard reactions with both organic and inorganic acids such as sulfuric, hydrochloric, phosphoric, acetic, succinic, citric, lactic, maleic, fumaric, cholic, pamoic, mucic, glutamic, camphoric, glutaric, glycolic, phthalic, tartaric, formic, lauric, stearic, salicylic, methanesulfonic, benzenesulfonic, sorbic, picric, benzoic, cinnamic and like acids

The term "carrier" is used herein to describe any ingredient other than the active components in a formulation. The choice of carrier will to a large extent depend on factors such as the particular mode of administration, the effect of the carrier on solubility and stability, and the nature of the dosage form.

Administration of spinetoram or a salt thereof may be systemically administered by any suitable route. Examples of suitable routes include oral, topical (transdermal), and parenteral administration. The choice of the route will depend on the species of the host animal and the nature of the ectoparasitic infestation. The administration will result in a systemic distribution within the host animal. Systemic efficacy (either by ingestion of blood by the parasites, or through a systemic repellant or in vivo action) provides a different mode of exposure as compared to non-systemically applied ectoparasiticides, where contact with the parasite at the skin surface is the mode of exposure. The advantages of systemic treatments and killing of parasites, compared to non-systemic treatments such as non-transdermal topical treatments, include: a) reduced exposure to the human applicator and children and objects in the animal's environment (*e.g*., flooring, carpets, furniture); b) no worry about loss from exposure of the animal to water (lakes, streams, bathing, *etc*.) or from loss due to rubbing; c) no concern about UV exposure and degradation; d) no problems with oxidation from oils on skin, *etc.;* and e) assurance that the entire dose is administered (compared to a topical, non-transdermal application where some of the dose may drip off, rub off and/or remain in the dispensing tube immediately after treatment).

Spinetoram and its salts may be formulated as pharmaceutical compositions for systemic administration. Such pharmaceutical compositions and processes for making the same are known in the art. *See, e.g.,* REMINGTON: THE SCIENCE AND PRACTICE OF PHARMACY, (A. Gennaro, et al., eds., 19th ed., Mack Publishing Co., 1995). Spinetoram or its salts may be present in the formulations in an amount of 1% to 90%, and more particularly, 5% to 60% by weight of the formulation.

The term "single-dose pharmaceutical formulation" means that one dose of the formulation effectively controls the ectoparasite infestation for a prolonged time. The term "prolonged time" comprises a period of at least 7 days, preferably a period of at least two weeks, and more preferably at least 30 day. The term "pulse dose formulation" means a formulation adapted for administration of a target total amount of spinetoram or its pharmaceutically acceptable salt in divided, distinct doses, normally administered over a short period of time such as a one or two day period. Pulse dosing is contrasted to single dosing in that while the therapeutic benefits are equal or substantially equivalent, the total dosing is carried out in more than one dosing over a short period of time. For instance, a total target dose may be pulse dosed by administering two, three, or four or more distinct, normally equal doses totaling the target dose over a one or two day period. Alternatively, a pulse dose may be accomplished by a single administration of the total target dose that is then released over time. This approach to pulse dosing can occur by having certain portions of the total dose released internally over time based on kinetics (*e.g*., every 2, 3, 4 or more hours) or based on location in the gastrointestinal tract (*e.g*., 50% in stomach, then 50% in the small intestine).

Oral administration may be by capsule, bolus, tablet, powders, lozenges, chews, multi and nanoparticulates, gels, solid solution, films, sprays, or liquid formulation. Liquid forms include suspensions, solutions, syrups, drenches and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet. Oral drenches are commonly prepared by dissolving or suspending the active ingredient in a suitable medium. Oral administration may be accomplished by admixing with, or placing on, an animal's food.

Spinetoram or its pharmaceutically acceptable salts may be administered to the skin, mucosa, or mucous membranes to result in a systemic administration. One such mode of administration is transdermal administration. Typical carriers include alcohol, water, mineral oil, liquid petrolatum, white petrolatum, glycerin, polyethylene glycol and propylene glycol. Penetration enhancers may be incorporated--see, for example, J. Pharm Sci, 88 (10), 955-958 by Finnin and Morgan (October 1999).

Further, spinetoram or its pharmaceutically acceptable salt can be administered parenterally, or by injection directly into the blood stream, muscle or into an internal organ. Suitable routes for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques. Injectable formulations may be prepared in the form of a sterile solution which may contain other substances, for example enough salts or glucose to make the solution isotonic with blood. Acceptable liquid carriers include vegetable oils such as sesame oil, glycerides such as triacetin, esters such as benzyl benzoate, isopropyl myristate and fatty acid derivatives of propylene glycol, as well as organic solvents such as pyrrolidin-2-one and glycerol formal. The formulations are prepared by dissolving or suspending spinetoram or its pharmaceutically acceptable salt in the liquid. These formulations may be self-preserving, self-sterilizing or may be non-sterile to which preservatives may be optionally added. Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a powdered or dried form to be used in conjunction with a suitable vehicle such as sterile, pyrogen-free water. The preparation of parenteral formulations under sterile conditions, for example, may readily be accomplished using standard pharmaceutical techniques well known to those skilled in the art. The solubility of spinetoram or its pharmaceutically acceptable salts used in the preparation of parenteral solutions may be increased by the use of appropriate formulation techniques, such as the incorporation of solubility-enhancing agents.

Spinetoram was evaluated using *in vitro* and *in vivo* bioassays to determine systemic activity. In many assays, spinosad was used as a comparator or a historical positive control, while other standards (fipronil, permethrin, imidacloprid) were employed. Spinetoram was employed both as technical active, as well as in formulation.

Adult Stable or House Fly Assay (ASF, AhsF). This assay is conducted essentially as described in White, W.H., S.M. Bauer, X. Zhao et al., Comparison of in vitro and in vivo ectoparasiticide activity of an experimental benzimidazole-carbamate with permethrin and amitraz, J. Med. Entomol. 42, 207-211 (2005); and White, W.H., C.M. McCoy, J.A. Meyer et al., Knockdown and mortality comparisons among spinosad-, imidacloprid-, and methomyl-containing baits against susceptible Musca domestica (Diptera: Muscidae) under laboratory conditions, J. Econ. Entomol. 100, 155-163 (2007).

Test material is formulated in DMSO at 10mM. Doubling dilutions in like solvent are made to yield 10 testing levels. Materials are diluted in either bovine serum (stable flies) or 5% glucose solution (house flies) to obtain desired exposure concentrations from 200-0.39 µM. Approximately 3ml of diluted test material is placed into a test tube (*n* = 3 per test level) and a dental wick is used to absorb fluid. One dental wick is placed into a small weigh boat inside of a 100mm Petri dish. Approximately 10 mixed-sex adult flies are anesthetized using carbon dioxide and counted into each dish. Dishes are incubated at 27°C and 50 - 70% relative humidity. Flies recover from anesthesia and feed on compound-soaked dental wicks. After 24 h, live/dead flies are enumerated. Nonlinear regression is used to model dose-mortality relationship and obtain relative potency (LD₅₀) data compared to contemporaneous controls (solvent-only or permethrin).

Table 1 below displays the summary of *in vitro* characterization for spinetoram (technical) versus standards against flies.

**Table 1**

| **Parasite** | **Compound** | **Potency (24 h EC₅₀ µM)** | **95% Cl of EC₅₀** | **Spinetoram/Spinosad Potency Ratio** |
|---|---|---|---|---|
| House Fly | Spinetoram | 2.178 | 1.732-2.740 | |
| | Spinosad | 11.96 | 9.452-15.13 | |
| | Fipronil | 0.9698 | 0.727-1.293 | 5.5 |
| | | | | |

Compared with spinosad, spinetoram exhibits significantly greater insecticidal activity *in vitro* against adult house flies (5.5 times more potent).

Oral administration per os to dogs. Given the above data, investigation into oral efficacy in dogs was begun. The investigation was conducted to evaluate 1) efficacy for a point dose of 30 mg/kg, against experimental, concurrent infestations with the adult cat flea, *Ctenocephalides felis;* adult stage American dog ticks, *Dermacentor variabilis;* and adult stage kennel ticks, *Rhipicephalus sanguineus;* 2) plasma concentrations of the 30 mg/kg treated dogs; and 3) efficacy for point dosages of either 50 mg/kg or 100 mg/kg, against experimental infestations with adult stage kennel ticks, *R. sanguineus.*

Sixteen dogs were selected for two groups of eight dogs (4 male: 4 female) per group. One treatment group received spinetoram, while the other group was left untreated. The dogs were housed individually in concrete-floored chain-link kennels both inside and outside. During the study period the dogs were fed a dry dog chow, except for the day of treatment (day 0) when they received wet canned food. The dogs had *ad libitum* access to water.

The first group on day 0 received one or more gelatin capsules containing spinetoram powder by mouth, in the amount of 30 mg/kg, while the untreated group received placebo. For the evaluation of the higher doses (50 and 100 mg/kg) for efficacy against *R*. *sangzuineus,* dogs from the 30 mg/kg group were re-dosed at the higher levels approximately 2.5 months after the low dose administration, once the flea efficacy data had been collected.

Each dog was experimentally infested with about 100 adult fleas, and 50 ticks of each species on test days -1, 5, 12, 19, 28, 35, and 42, with additional flea infestations conducted on days 49 and 56. Knockdown assessments against both ticks and fleas were conducted 24 hours after dosing. All subsequent counts were conducted 48 hours after infestation. For the higher doses, the dogs were infested with around 50 *R. sanguineus* ticks one day before re-dosing and again 5 days post re-dosing, with comb counts conducted approximately 24 hours thereafter. Knockdown activity (Day 1) was determined using 24 hr post-dosing comb counts. For all post-treatment residual tick counts, the comb count occurred 48 hours post infestation. Blood samples were drawn on days 14, 21, 28, and 35 to determine concentration of spinetoram in plasma.

Table 2 displays the therapeutic and residual efficacy for fleas (geometric mean percent flea reduction) of spinetoram following oral administration to dogs at 30 mg/kg.

**Table 2**

| **Material** | **knockdown¹** | **Day 7** | **Day 14** | **Day 21** | **Day 30** | **Day 37** | **Day 44** | **Day 51** | **Day 58** |
|---|---|---|---|---|---|---|---|---|---|
| Spinetoram | 99.7 | 100 | 100 | 99.5 | 100 | 98.9 | 99.7 | 98.2 | 96.9 |
| Spinosad² | 100 | 100 | 99.6 | 100 | 97.8 | 91.1 | nd³ | nd | nd |

| | | | | | | | | | |
|---|---|---|---|---|---|---|---|---|---|
| ¹ Knockdown assessed 48 h after dosing. ² Represents historical data and not contemporaneous controls. ³ nd, denotes not determined. | | | | | | | | | |

At an oral point dose of 30 mg/kg in dogs, spinetoram exhibited equivalent knockdown and superior residual efficacy when compared to historical data of spinosad against adult cat flea infestations. Note the residual control (≥97%) extends beyond 8 weeks. Spinetoram did not demonstrate statistically significant activity against either of the tick species at the doses tested. In general, efficacy of the treatments against both tick species was difficult to interpret because of very low parasite retention numbers on the untreated control animals.

Pulse vs. Single dose administration per os to dogs/*Ctenocephalides felis* Another dog study was undertaken to evaluate the effect on flea infestation of 1) oral administration of a single point dose of 60 mg/kg; 2) oral administration of a pulsed dosing scheme of 20 mg/kg TID every 2 hours for one day; 3) oral administration of a pulsed dosing scheme of 20 mg/kg TID every 4 hours for one day; and 4) post-treatment plasma concentration of spinetoram.

Four treatment groups of 8 dogs each (4 male: 4 female) were dosed spinetoram as follows:
Treatment Group 1: 60 mg/kg, single dose
Treatment Group 2: 60 mg/kg, (20 mg/kg every 2 hours, three times)
Treatment Group 3: 60 mg/kg, (20 mg/kg every 4 hours, three times)
Treatment Group 4: 0 mg/kg, (negative control)

The dogs were housed individually in concrete-floored chain-link kennels both inside and outside. During the study period the dogs were fed a dry dog chow, except for two days prior to and the day of treatment (day 0) when they received wet canned food. The dogs had *ad libitum* access to water.

The treated dogs received one or more gelatin capsules containing technical active spinetoram powder by mouth, with treatment group 4 receiving placebo. Each dog was experimentally infested with about 100 fleas on test days -1, 5, 12, 19, 28, 35, 42, 49, and 56. Knockdown assessments were conducted 24 hours after dosing, with residual efficacy evaluated subsequently at 48 hours after each subsequent infestation. The day 1 comb count served to determine the initial/knockdown efficacy, approximately 24 hours after the 60mg/kg dose, or the first of the pulse dose. Blood samples were drawn to determine concentration of spinetoram in plasma.

Table 3 shows the geometric mean percent reduction in live adult flea counts compared to the untreated control group. Results indicate no substantial difference in the efficacy results whether a single 60 mg/kg dose or multiple 20 mg/kg doses are administered.

**Table 3**

| | **Day 1** | **Day 7** | **Day 14** | **Day 21** | **Day 30** | **Day 37** | **Day 44** | **Day 51** | **Day 58** | **Day 72** | **Day 86** | **Day 93** | **Day 100** | **Day 107** |
|---|---|---|---|---|---|---|---|---|---|---|---|---|---|---|
| **Untreated** | 76.3 (---) | 75.5 (---) | 86.1 (---) | 79.7 (---) | 80.6 (---) | 75.1 (---) | 78.2 (---) | 68.1 (---) | 79.7 (---) | 88.0 (---) | 76.6 (---) | 75.1 (---) | 83.2 (---) | 81.9 (---) |
| **60 mg/kg SID** | 0.0 (100) | 0.0 (100) | 0.0 (100) | 0.0 (100) | 0.0 (100) | 0.0 (100) | 0.0 (100) | 0.0 (100) | 0.2 (99.8) | 0.0 (100) | 1.8 (97.7) | 1.9 (97.5) | 5.2 (93.7) | 3.2 (96.2) |
| **20 mg/kg TID@2h** | 0.0 (100) | 0.0 (100) | 0.0 (100) | 0.0 (100) | 0.0 (100) | 0.0 (100) | 0.0 (100) | 0.0 (100) | 0.0 (100) | 0.0 (100) | 6.4 (91.7) | 2.5 (96.7) | 10.0 (88.0) | 4.8 (94.1) |
| **20 mg/kg TID@4h** | 0.0 (100) | 0.0 (100) | 0.0 (100) | 0.0 (100) | 0.2 (99.8) | 0.0 (100) | 0.0 (100) | 0.2 (99.7) | 0.0 (100) | 0.2 (99.8) | 4.7 (93.8) | 4.8 (93.7) | 11.4 (86.3) | 8.5 (89.6) |

## Claims

1. Spinetoram, or a pharmaceutically acceptable salt thereof, for use in controlling flea infestations on a dog or cat, wherein the spinetoram is to be systemically administered by oral administration.

2. The spinetoram, or a pharmaceutically acceptable salt thereof, for use according to claim 1, wherein said flea is *Ctenocephalides felis.*

3. The spinetoram, or a pharmaceutically acceptable salt thereof, for use according to claim 1, wherein said administration is to be carried out no more than every two weeks in a single or pulse dose.

4. The spinetoram, or a pharmaceutically acceptable salt thereof, for use according to claim 3, wherein said administration is to be carried out no more than monthly.

5. The spinetoram, or a pharmaceutically acceptable salt thereof, for use according to claim 1, wherein the amount to be administered is 10 mg/kg to 60 mg/kg of body weight of said dog or cat.

6. A single or pulse dose formulation for systemically controlling ectoparasite infestations on a dog or cat comprising spinetoram, or a pharmaceutically acceptable salt thereof, and a pharmaceutically acceptable carrier in an oral dosage form selected from a tablet, capsule or liquid, at a dose of 10 to 60 mg of said spinetoram or a pharmaceutically acceptable salt thereof per kg of body weight of said dog or cat.

7. The formulation of claim 6, wherein the dosage form is a tablet or capsule and said spinetoram, or a pharmaceutically acceptable salt thereof, is present in said formulation in an amount of 5 to 60 % by weight of the formulation.

8. The formulation of claim 6 in a chewable treat form.

## Patentansprüche

1. Spinetoram oder ein pharmazeutisch akzeptables Salz hiervon zur Verwendung bei der Bekämpfung eines Flohbefalls bei einem Hund oder einer Katze, wobei das Spinetoram durch orale Verabreichung systemisch zu verabreichen ist.

2. Spinetoram oder ein pharmazeutisch akzeptables Salz hiervon zur Verwendung nach Anspruch 1, wobei es sich bei den Flöhen um *Ctenocephalides felis* handelt.

3. Spinetoram oder ein pharmazeutisch akzeptables Salz hiervon zur Verwendung nach Anspruch 1, wobei die Verabreichung nicht mehr als alle zwei Wochen in einer einzelnen Dosis oder Pulsdosis durchzuführen ist.

4. Spinetoram oder ein pharmazeutisch akzeptables Salz hiervon zur Verwendung nach Anspruch 3, wobei die Verabreichung nicht mehr als einmal monatlich durchzuführen ist.

5. Spinetoram oder ein pharmazeutisch akzeptables Salz hiervon zur Verwendung nach Anspruch 1, wobei die zu verabreichende Menge 10-60 mg/kg Körpergewicht des Hundes oder der Katze beträgt.

6. Einzeldosis- oder Pulsdosisformulierung zur systemischen Bekämpfung eines Ektoparasitenbefalls bei einem Hund oder einer Katze, die Spinetoram oder ein pharmazeutisch akzeptables Salz hiervon und einen pharmazeutisch akzeptablen Träger umfasst, in einer oralen Dosierungsform, die aus einer Tablette, Kapsel oder Flüssigkeit ausgewählt ist, in einer Dosis von 10 - 60 mg des Spinetorams oder eines pharmazeutisch akzeptablen Salzes hiervon pro Kilogramm Körpergewicht des Hundes oder der Katze.

7. Formulierung nach Anspruch 6, wobei die Dosierungsform eine Tablette oder Kapsel ist und wobei das Spinetoram oder ein pharmazeutisch akzeptables Salz hiervon in der Formulierung in einer Menge von 5 - 60 Gew.-% der Formulierung vorhanden ist.

8. Formulierung nach Anspruch 6 in einer kaubaren Behandlungsform.

## Revendications

1. Spinetoram, ou un de ses sels pharmaceutiquement acceptables, pour son utilisation destinée à juguler des infestations par une puce sur un chien ou sur un chat, le spinetoram devant être administré par voie systémique par administration orale.

2. Spinetoram, ou un de ses sels pharmaceutiquement acceptables, pour son utilisation selon la revendication 1, dans lequel ladite puce est *Ctenocephalides felis.*

3. Spinetoram, ou un de ses sels pharmaceutiquement acceptables, pour son utilisation selon la revendication 1, dans lequel ladite administration ne peut pas être mise en oeuvre plus d'une fois toutes les deux semaines en une dose unique ou pulsée.

4. Spinetoram, ou un de ses sels pharmaceutiquement acceptables, pour son utilisation selon la revendication 3, dans lequel ladite administration ne peut pas être mise en oeuvre plus d'une fois par mois.

5. Spinetoram, ou un de ses sels pharmaceutiquement acceptables, pour son utilisation selon la revendication 1, dans lequel la quantité à administrer s'élève de 10 mg/kg ou 60 mg/kg du poids corporel dudit chien ou dudit chat.

6. Formulation sous la forme d'une dose unique ou pulsée pour la jugulation systémique d'infestations par un ectoparasite sur un chien ou sur un chat comprenant du spinetoram ou un de ses sels pharmaceutiquement acceptables et un support pharmaceutiquement acceptable dans une forme posologique orale choisie parmi un comprimé, une capsule ou un liquide, à une dose de 10 à 60 mg dudit spinetoram ou d'un de ses sels pharmaceutiquement acceptables par kg du poids corporel dudit chien ou dudit chat.

7. Formulation selon la revendication 6, dans laquelle la forme posologique est un comprimé ou une capsule et ledit spinetoram ou un de ses sels pharmaceutiquement acceptables est présent dans ladite formulation en une quantité de 5 à 60 % en poids de la formulation.

8. Formulation selon la revendication 6, sous une forme de traitement à croquer.
